# EUROPEAN PATENT SPECIFICATION

(11) **EP 2 108 342 B1**
(45) Date of publication and mention of the grant of the patent: **07.07.2010**
(21) Application number: 09007341.2
(22) Date of filing: 15.11.2007
(51) Int. Cl.: A61F 2/82, A61M 31/00

(54) **Medical devices for localized drug delivery**
Medizinische Vorrichtungen zur lokalen Arzneimittelabgabe
Dispositifs médicaux pour l'administration localisée de médicaments

(30) Priority: 07.12.2006 US 873481 P
(43) Date of publication of application: 14.10.2009
(62) Divisional of application: 07862071.3
(73) Proprietor: Mallinckrodt Inc., Hazelwood, MO 63042 (US)
(72) Inventor: Cantrell, Gary L., Illinois 62294 (US); Berberich, David W., St. Peters Missouri 63376 (US)
(74) Representative: Chettle, Adrian John

(56) References cited:
- WO-A-00/18331
- GB-A- 2 352 635
- US-A1- 2002 182 750
- US-A1- 2005 238 689
- US-B1- 6 491 617

## Description

### FIELD OF THE INVENTION

The present invention relates to implantable medical devices for localized drug delivery.

### BACKGROUND

It is now commonplace for a stent, such as a coronary stent, to be coated with a drug for localized delivery to surrounding tissue once the device is implanted in a patient. These types of stents are called drug-eluting stents. One type of drug-eluting stent releases over time an anti-restenosis drug for preventing restenosis of the coronary wall in and nearby the supporting stent. The stent typically comprises a metallic framework formed into a tubular body. The body is coated with a polymer that is loaded, i.e., impregnated, with the drug. The polymer may be biodegradable, whereby the drug is released from the polymer as the polymer degrades.

One problem associated with drug-eluting stents is the possibility of the patient having an allergic reaction to the polymer or polymer metabolites because the polymer of the stent is exposed to the patient's circulatory system, and thus the patient's immune system. It is also possible that the patient may have an allergic reaction to the eluted drug or otherwise have a negative biological response to the drug. It is typically not known if the patient will be allergic to the polymer and/or the drug until after the patient begins suffering symptoms of the allergic reaction. In some cases, the allergic reaction can lead to anaphylactic shock, during which the patient will have difficulty breathing and low blood pressure and may have a cardiac arrest. Although medication, such as an antihistamine or cortisone, a severe reaction may require surgical removal of the drug-eluting stent.

Another problem associated with conventional drug-eluting stents is the possibility that the eluted drug will not be effective. For example, if an anti-restenosis drug is not effective and restenosis is developing, then the patient may have to undergo bypass surgery. Such surgery involves higher risk and increased cost and recovery time for the patient.
WO-A-00/18331 discloses an implantable medical device having the features of the pre-characterizing portion of claim 1. US-B1-6491617 discloses a medical device having a bio-compatible material with exogenous storage structures for a therapeutic agent.

### SUMMARY

Certain aspects of the invention are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

According to the invention there is provided an implantable medical device, comprising:
an elongate member configured in a plurality of circular turns forming a first helix, wherein the first helix defines an internal cavity, and wherein adjacent turns of the first helix define an opening connecting the internal cavity with the outside of the helix; and,
a polymeric structure disposed within the internal cavity, **characterized in that** said polymeric structure has a plurality of artificial first receptor site mimics, and said opening is sized sufficiently to prevent blood cells from entering the internal cavity.

In some embodiments of the invention, one or more receptor sites are located in the internal cavity. The one or more sites may be receptor adapted to repeatedly bind to, temporarily hold, and release an active agent. One or more openings extend from the internal cavity through the body of the device. The one or more openings are sized so that after the medical device is implanted in the patient, any active agent present in the patient's bloodstream may enter the internal cavity through the opening to bind to the receptor sites and exit the internal cavity through the opening when released from the receptor sites.

A plurality of openings extending from the internal cavity through the body may be distributed over the body of the device. These openings are sized and shaped to substantially prevent blood cells from entering the internal cavity while allowing the active agent to exit the internal cavity through the openings.

The body of the device includes an elongate, helical member in which the internal cavity is defined. Adjacent turns of the helical member are spaced apart to define at least one opening for allowing the active agent to exit the internal cavity between the adjacent turns.

The device of the invention may be used for delivering an active agent from an implantable medical device for purposes of medically treating a patient. In this aspect, the medical device is implanted into the patient. The implanted medical device is systemically loaded with an active agent so that the active agent enters an internal cavity of the device through an opening in the device and binds to receptor sites within the internal cavity that are adapted to bind to and release the active agent. The active agent is allowed to be released from the receptor sites of the device so that the active agent travels out of the medical device and into surrounding tissue.

In another aspect the invention is directed to a method of making an implantable medical device. In this aspect, a body of the device is formed so that it has an internal cavity and a plurality of openings extending from the cavity through the body. A polymer having receptor site mimics for an active agent is formed. The receptor site mimics are each adapted to receive and temporarily hold an active agent and to release the active agent. The polymer is disposed in the internal cavity of the body.

The device may be used to treat a patient with a stenotic artery. In this aspect, a stent is implanted into the stenotic artery to open the artery. The stent includes a body, as well as a plurality of receptor site mimics associated with the body and adapted to receive and temporarily hold an active agent and to release the active agent. At least after implantation, the artery is monitored for restenosis, and the active agent is introduced into the patient's bloodstream if the artery is detected as being restenotic. Subsequent to being introduced into the bloodstream, the active agent temporarily binds to the receptor site mimics of the stent.

The device may be loaded with an active agent after the device has been implanted into a patient. In this aspect, the active agent is introduced into the patient's bloodstream. The active agent in the bloodstream enters into an internal cavity in the medical device through at least one opening in the device and is temporarily captured in the internal cavity of the medical device. Further, the cells of the patient's immune system are prevented from entering the internal cavity of the medical device through the opening therein.

Various refinements exist of the features noted above in relation to the various aspects of the present invention. Further features may also be incorporated in these various aspects as well. These refinements and additional features may exist individually or in any combination. For instance, various features discussed below in relation to one or more of the illustrated embodiments may be incorporated into any of the above-described aspects of the present invention alone or in any combination. Again, the brief summary presented above is intended only to familiarize the reader with certain aspects and contexts of the present invention without limitation to the claimed subject matter.

### BRIEF DESCRIPTION OF THE FIGURES

Fig. 1 is a perspective of a stent including a member formed into a helical body of the stent;

Fig. 2 is an enlarged fragment of the member of Fig. 1, with a portion of the member broken away to schematically show polymeric structures that are disposed in the member;

Fig. 3 is an enlarged, schematic fragment of one of the polymeric structures of Fig. 2 illustrating an artificial receptor site mimic and an active agent binding to the receptor site mimic;

Fig. 4 is another example of a stent having two different types of polymeric structures with two different artificial receptor site mimics, the polymeric structures being enlarged in a detail to show the two artificial receptor site mimics and two corresponding active agents binding to the respective receptor site mimics;

Fig. 5 shows a stent including framework members in a lattice configuration;

Fig. 6 is an embodiment of a stent including a helical member formed into a helical body of the stent; and

Fig. 7 is an enlarged fragmentary section of the helical member of Fig. 6.

Corresponding reference characters indicate corresponding parts throughout the figures.

### DEATILED DESCRIPTION OF EXEMPLARY EMBODIMENTS

One or more specific embodiments of the present invention are described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

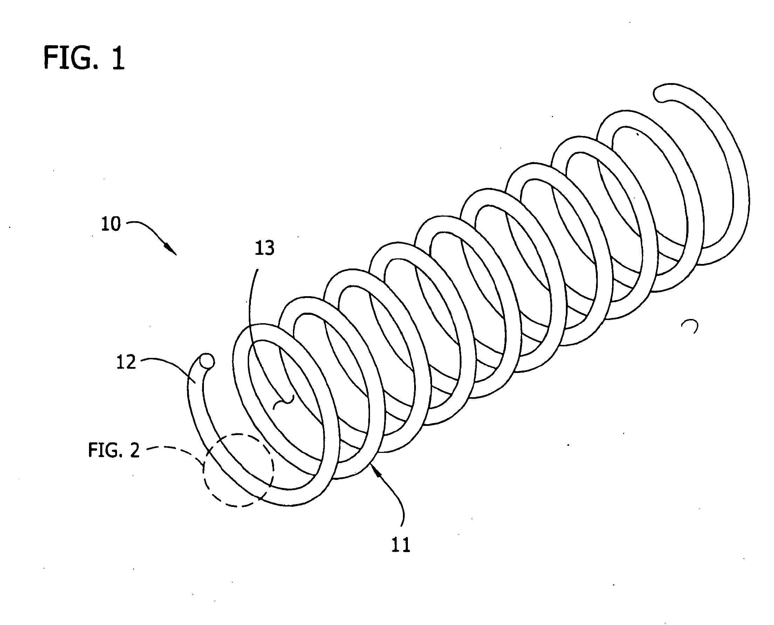

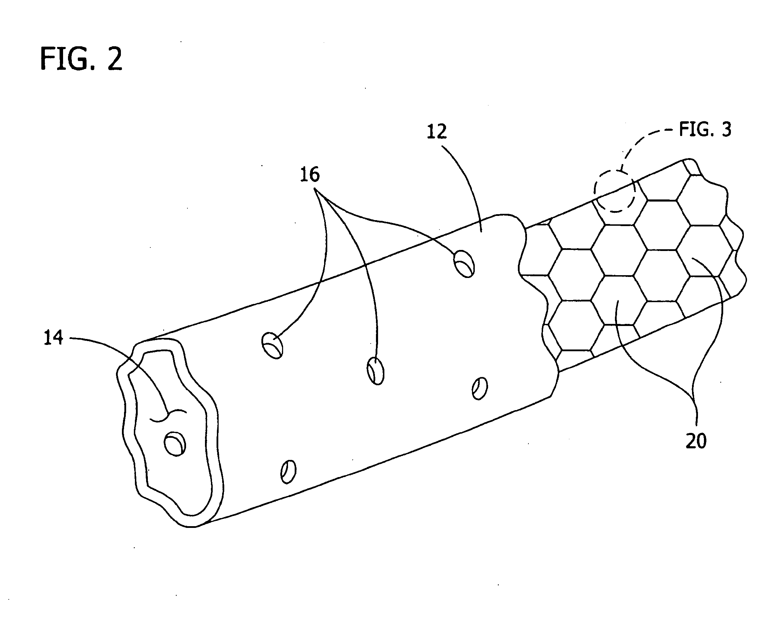

Referring to Figs. 1 and 2, a cardiovascular stent (broadly, a medical device) is generally indicated at 10. A body of the stent, generally indicated at 11, is formed by an elongate member 12 that wound into a helix so that the body of the stent includes a central passage 13 that allows blood to flow through the stent when it is implanted. Other ways of forming the stent do not depart from the scope of the present invention. In an initial configuration, the body 11 of the stent 10 is sized and shaped to be received on and around a deflated balloon of a balloon catheter (or other suitable catheter). The general structure and function of a balloon catheter is well known in the art and therefore will not be described in detail. Briefly, during an implantation procedure, the catheter is used to guide the stent 10 on the deflated balloon through the vasculature of the patient to the patient's coronary artery. With the balloon and stent properly positioned in the artery, the balloon is inflated and the helical stent 10 expands radially to an expanded configuration. The balloon is then deflated and the helical stent 10 remains in the expanded configuration. The catheter is then removed, leaving the expanded stent in the artery to hold the artery open. The stent 10 may be constructed of Nickel-Titanium (NiT), surgical stainless steel or other suitable materials.

Referring to Figs. 1-4, the helical elongate member 12 of the stent 10 has an internal cavity 14 extending along its length and a plurality of openings 16 extending from the internal cavity 14 of the member 12 to outside the member. The openings 16 are distributed around an entire exterior surface of the member 12. In the embodiment of Figs. 1-4, the openings 16 are spaced apart from each other and spread substantially uniformly over the length and circumference of the member 12. Other arrangements of the openings, including non-uniform distributions are contemplated as being within the scope of the present invention. As explained in more detail below, an active agent 18 is disposed within the internal cavity 14 of the member 12. The openings 16 can be desirably sized and shaped to allow the active agent 18 to exit the internal cavity 14 of the member 12 while substantially preventing blood cells from entering the member 12. Each opening preferably has a diameter less than or equal to about 8.0 microns to substantially prevent blood cells, more specifically white blood cells (i.e., leukocytes), from entering the member.

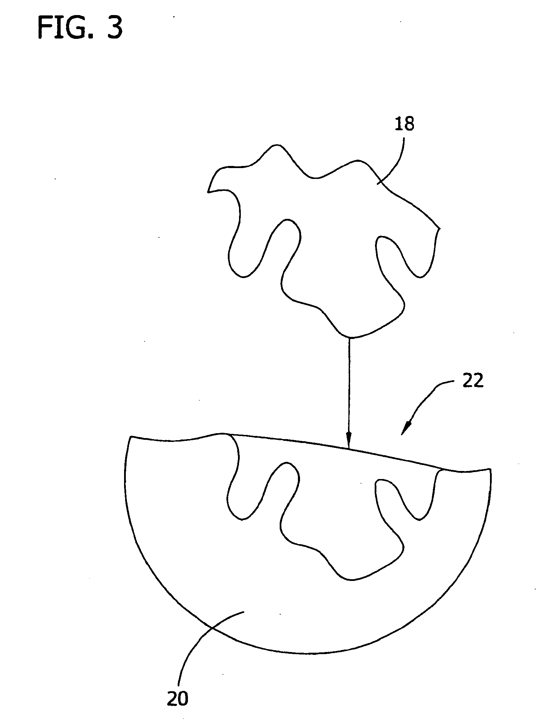

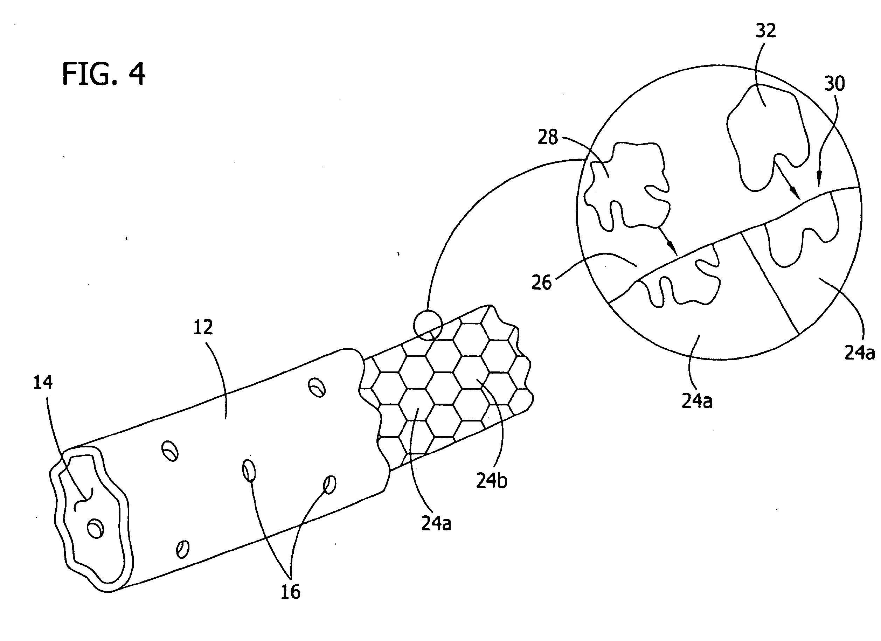

A plurality of polymeric structures 20 is disposed in the internal cavity 14 of the member 12. It will be understood that the polymeric structures 20 can be disposed elsewhere on the stent 10 within the scope of the present invention. The polymeric structures 20 have one or more receptor site mimics 22, one of which is generally indicated at 22 (Fig. 3), for binding the active agent 18 and temporarily holding active agent in the internal cavity 14. The receptor site mimics 22 can be artificially created receptor sites having a pre-selected affinity for the active agent 18. In other words, the receptor site mimics 22 act like a biological receptor site, and therefore, the polymeric structures 20 are capable of being loaded and reloaded with the active agent 18. The receptor site mimics 22 may be formed by molecular imprinting. Molecular imprinting involves imprinting a molecule, such as the active agent 18, on a polymeric substrate or template, such as the polymeric structures 20, so that that the imprint on the polymeric substrate is of the three-dimensional shape of the molecule. In this way, the imprint acts as a receptor for binding to the molecule (Fig. 4). Ways of molecularly imprinting polymers are known in the art and will not be discussed in detail herein. It is understood that the polymeric structures 20 may be formed as beads and/or nano-spheres or may be other shapes. Moreover, a single polymeric structure having the molecular imprints (i.e., artificial receptor site mimics) may be disposed in the internal cavity 14 of the member 12 within the scope of the present invention. Other ways of making receptor site mimics are within the scope of this invention.

Based on the affinity of the receptor site mimics 22, the polymeric structures 20 may hold the active agent in the internal cavity 14 for a certain period or certain range of time before the active agent becomes detached from the mimics. The receptor site mimics 22 may be constructed to have varying affinities so that the active agent 18 is released from the stent 10 at a controlled rate. For example and without limitation, a pre-selected number of the receptor site mimics 22 may have a relatively weak affinity for the active agent 18 and will release the active agent after holding it for between about 30 minutes and about 1 hour. Another number of the receptor site mimics 22 may have a stronger affinity for the active agent 18 and will release the active agent after holding it for between about 1 hour and about 2 hours. Thus, the active agent 18 is continuously released from stent 10 at a controlled rate after it has been loaded, as opposed to releasing the active agent as a bolus. It is understood that the receptor site mimics 22 may all have the same affinity for the active agent 18 so that the active agent is released as a bolus.

After the stent 10 is implanted in the patient, it may be reloaded (or initially loaded) systemically with the active agent 18. That is, the active agent 18 may be introduced into the patient's bloodstream and allowed to enter the internal cavity 14 of the member 12 via the micron-sized openings 16 and bind to the receptor site mimics 22 of the polymeric structures 20 (Fig. 4). For example, the patient may ingest the active agent 18 (i.e., in pill form) or the agent may be delivered intravenously. Other ways of introducing the active agent 18 into the bloodstream, including via the respiratory system, is within the scope of this invention. Other ways of loading the internal cavity 14 of the member 12 are within the scope of this invention.

As discussed above, the micron-sized openings 16 preclude white blood cells from entering the internal cavity 14 but allow the active agent to enter from the bloodstream. Preferably, the micron-sized openings 16 preclude T-cells and B-cells (broadly, lymphocytes) and basophil cells (broadly, granulocytes), as each of these white blood cells play a major role in producing an allergic condition and an allergic reaction in the blood. Because each of these types of blood cells are about 8.0 microns or larger in diameter, the openings 16 preferably have diameter less than about 8.0 microns, more preferably less than about 5 microns, and more preferably between about 0.5 microns and about 1.0 microns, to isolate the polymeric structures 20 from the patient's bloodstream and immune system to prevent any negative biological responses (e.g., allergic reactions) caused by the polymeric structures.

Other ways of preventing blood cells, especially white blood cells, from entering the internal cavity via the openings 16 are within the scope of the invention. For example, the exterior surface of the stent 10 or a coating on the exterior surface may be polarized. In one embodiment, a coating on the exterior surface may be loaded with albumin, which effectively polarizes the surface. By being polar, blood cells are repelled away from the exterior surface of the stent 10 because blood cells are themselves polar. Alternatively, portions of the stent 10 defining the openings 16 may be polarized or coated with a polar material to repel blood cells away from only the openings. Other ways of preventing blood cells from entering the internal cavity are within the scope of the invention.

In one example, the active agent 18 that is pre-loaded and/or systemically loaded in the stent 10 comprises an anti-restenosis drug for preventing restenosis of the artery in which the stent is implanted. The anti-restenosis drug may be an anti-proliferative agent that prevents the proliferation of the surrounding tissue, e.g., vascular endothelial cells. For example, the active agent may be either a microtubule stabilizer, such as paclitaxel, a taxane, or a microtubule destabilizer, such as vincristine, vinblastine, podophylotoxin, estramustine, noscapine, griseofulvin, dicoumarol, and a vinca alkaloid. A microtubule stabilizer operates to enhance microtubule polymerization which inhibits cell replication by stabilizing microtubules in spindles which block cell division. The microtubule destabilizer promotes microtubule disassembly to prevent cell replication.

In another example, the active agent 18 may comprise, in addition to or as an alternative to the anti-restenosis drug, an anti-thrombosis drug, such as an anticoagulant or anti-clotting agent, for reducing the possibility of thrombosis in and around the stent 10. For example, the anti-clotting agent may be a heparin, preferably a low molecular weight heparin or heparinoid. Other active agents may also be used in conjunction with the anti-thrombosis drugs, such as Warfarin, RGD peptides and aspirin.

In an exemplary use, the stent 10 (loaded with the active agent 18 or unloaded) is implanted in the coronary artery of the patient, as briefly described above. The active agent 18 is then introduced into the patient's bloodstream to load (or reload) the stent 10. The timeline for introducing the active agent 18 into the patient's bloodstream depends on whether the polymeric structures 20 were pre-loaded and the type of active agent. The active agent 18 is allowed to be released from the stent 10 and into the arterial wall of the coronary artery. The active agent is then introduced into the patient's bloodstream to reload the stent. The reloading procedure may be repeated as necessary. If restenosis does not occur, then no active agent may ever be introduced.

In another exemplary use, the stent 10 may be implanted in the coronary artery of the patient without being loaded with the active agent 18. The patient is monitored to determine if restenosis of the coronary artery is developing. If it is determined that restenosis is occurring, then the active agent 18 is introduced into the patient's bloodstream where it enters the internal cavity 14 of the member 12 and binds to the receptor site mimics 22 of the polymeric structures 20. The active agent 18 is the allowed to exit the stent 10 for localized delivery to the arterial wall of the coronary artery. The reloading procedure may be repeated as necessary.

It is contemplated that the stent 10 may not be reloadable, but instead may be pre-loaded (i.e., before implantation) with a finite amount of active agent 18. For example, a polymer or other type of matrix that is biodegradable may be loaded with the active agent 18. The active agent 18 is released into the surrounding tissue via the openings 16 as the polymer degrades. The polymer is isolated from the patient's bloodstream to prevent any negative responses due to the polymeric material. Other ways of pre-loading and releasing the active agent from the internal cavity of the frame member is within the scope of this invention.

The internal cavity 14 of the stent 10 may be pre-loaded and/or systemically loaded with two or more different active agents. Referring to Fig. 4, this stent can be similar to what has been already described except that first polymeric structures 24a disposed in the internal cavity 14 have one or more first receptor site mimics 26 for binding a first active agent 28 and second polymeric structures 24b disposed in the internal cavity have one or more second receptor site mimics 30 for binding a second, different active agent 32. It is understood that the stent 10 may have other polymeric structures having number of different types of receptor site mimics for binding to any number of different active agents. Moreover, each polymeric structure 24a, 24b may have one or more types of receptor site mimics. For example, each polymeric structure may have first receptor site mimics 26 and second receptor site mimics 30. The two or more different active agents may be systemically loaded in a different way and that the stent may instead be pre-loaded in other ways, such as described above.

In one exemplary use of the stent 10 with the polymeric structures 24a, 24b of Fig. 4, the stent may be loaded with the two active agents 28, 32 simultaneously so that the active agents are released into the tissue simultaneously.. Simultaneous treatment with two or more different active agent may have a synergistic effect for preventing restenosis. For example, the first active agent 28 may be a microtubule stabilizer, such as paclitaxel, and the second active agent 32 may be a microtubule destabilizer, such as noscapine. These active agents 28, 32 may be loaded in the stent 10 and delivered simultaneously to the coronary wall after angioplasty to prevent restenosis. In this example, the paclitaxel, which is shown to be toxic in high doses, may be delivered in a lesser dose than typically needed when it is supplemented simultaneously with the less toxic noscapine.

In another example, the stent 10 with the polymeric structures 24a, 24b of Fig. 4, may be initially loaded (i.e., either pre-loaded or systemically loaded) with one type of active agent. For example, the stent 10 may be initially loaded with only the first active agent 28. If complications arise with the initial active agent 28, such as an allergic reaction, or if the initial active agent is determined to be ineffective, the treatment can be switched to another active agent (e.g., the second active agent 32) that is capable of being systemically loaded in the stent. Depending on the number of different type of receptor site mimics, the second active agent 32 can be replaced, and so on, if any previous active agent is problematic or ineffective. Thus, alternative treatments can be given to the patient without having to remove the stent 10.

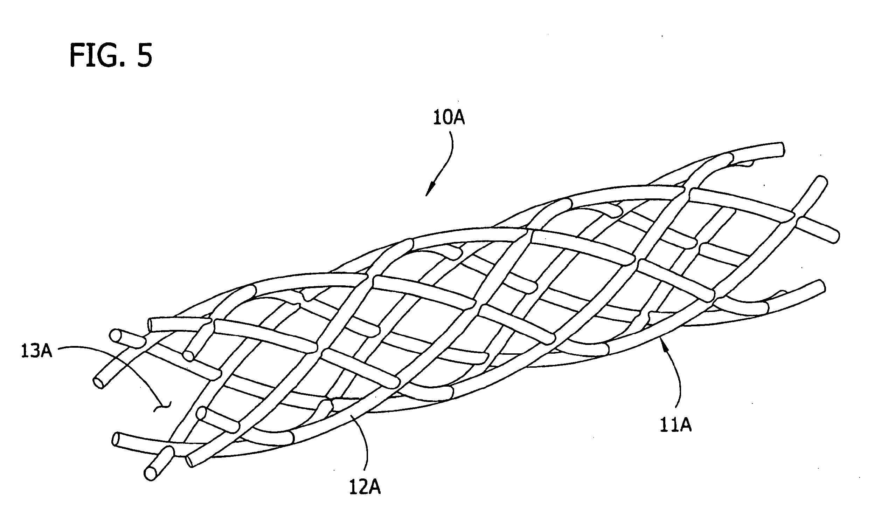

Fig. 5 illustrates a lattice stent 10A having a lattice framework that is configured into a generally tubular body 11A of the stent. The lattice framework comprises framework members 12A that are analogous to the member 12 of the helical stent 10. These framework members 12A can have internal cavities and a plurality of openings extending radially from the internal cavity of each framework member to outside the framework member. The internal cavities may be loaded (pre-loaded or loaded systemically), as described above. Other constructions are possible.

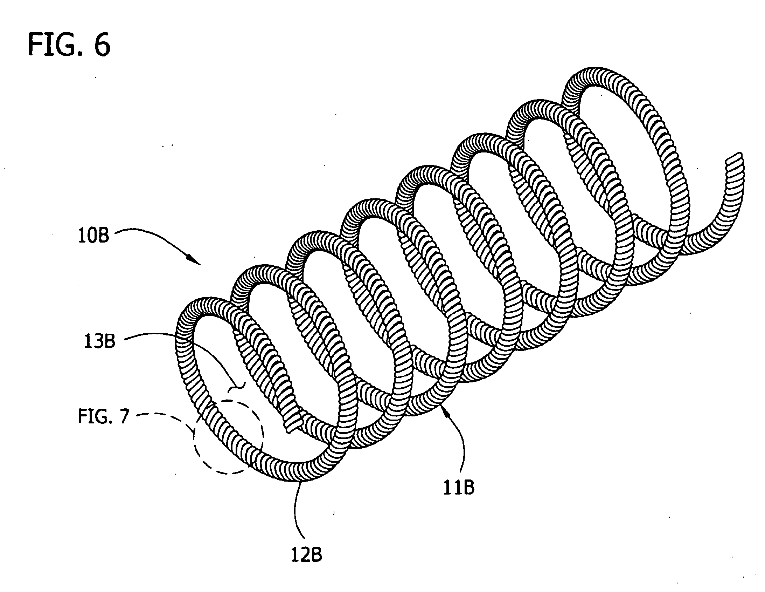

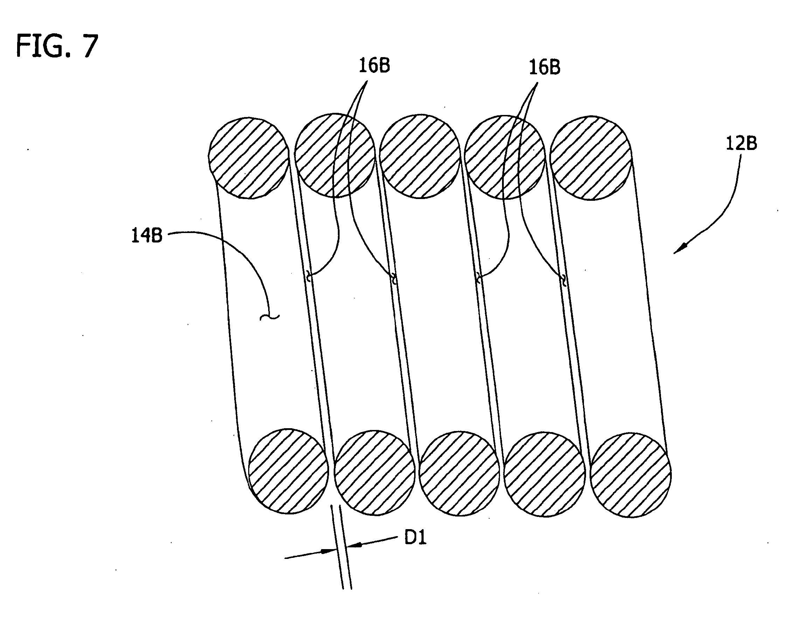

Referring to Figs. 6 and 7, a stent according to the invention is generally indicated at 10B. The stent includes a member 12B formed into a helix (broadly, a first helix) defining an internal cavity 14B, similar to the internal cavity 14 of previous examples. The helical member 12B is formed into a helical body 11B (broadly, a second helix) that defines a central passage 13B, similar to the central passage 13 of previous examples. Thus, the stent 10B may be described as a helical helix. Like previous examples, the internal cavity 14B may be loaded (i.e., pre-loaded and/or systemically loaded) with an active agent(s) as described (e.g., using polymeric structures having receptor site mimics). Openings 16B in the helical member 12B (i.e., in the first helix) are formed by adjacent turns of the member spaced apart a distance D1 to allow for the active agent 18 to be released from the internal cavity 14B and for the active agent to enter the internal cavity while substantially preventing blood cells from entering the internal cavity. The distance D1 between adjacent turns of the helical member 12B is preferably no greater than about 8 microns, more preferably less than about 5 microns, more preferably between about 0.5 microns and about 1.0 microns. The openings 16B in the member 12B can be considered a single helical opening.

It is contemplated that the present invention may be directed to other stents besides coronary stents and to other medical devices aside from stents. That is, the body of the medical device does not have to take on a configuration like that of the stent. Thus, this medical device is essentially the member 12 not configured into a specific shape or form.

Having described the invention in detail, it will be apparent that modifications and variations are possible without departing from the scope of the invention defined in the appended claims.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

While the invention may be susceptible to various modifications and alternative forms, specific embodiments have been shown by way of example in the drawings and have been described in detail herein. However, it should be understood that the invention is not intended to be limited to the particular forms disclosed. Rather, the invention is to cover all modifications, equivalents, and alternatives falling within the scope of the invention as defined by the following appended claims.

## Claims

1. An implantable medical device (10), comprising:
an elongate member (12B) configured in a plurality of circular turns forming a first helix, wherein the first helix defines an internal cavity (14B), and wherein adjacent turns of the first helix define an opening (16B) connecting the internal cavity (14B) with the outside of the helix; and,
a polymeric structure (20) disposed within the internal cavity (14), **characterized in that** said polymeric structure (20) has a plurality of artificial first receptor site mimics (22), and said opening (16) is sized sufficiently to prevent blood cells from entering the internal cavity (14).

2. The device (10) of claim 1, wherein the first helix is further configured to form a second helix (11B), the second helix defining a central passage (13B) for passage of fluid therethrough.

3. The device (10) of claim 1 or claim 2, wherein the said opening (16B) is defined by a space (D1) between adjacent turns of the first helix.

4. The device (10) of claim 3, wherein the space (D1) between adjacent turns of the first helix is less than or equal to about 8.0 microns.

5. The device (10) of claim 4, wherein the space (D1) between adjacent turns of the first helix is less than about 5 microns.

6. The device (10) of claim 5, wherein the space (D1) between adjacent turns of the first helix is between about 0.5 microns and about 1.0 microns.

7. The device (10) of any preceding claim, wherein the first receptor site mimics (22) are each configured to bind a first active agent (18).

8. The device (10) of claim 7, wherein the first receptor site mimics (22) are defined in the polymeric structure (20) by a molecular imprint of the active agent (18).

9. The device (10) of any preceding claim, further comprising a plurality of second receptor site mimics (22), wherein each second receptor site mimic (22) is configured to bind a second active agent (32).

10. The device (10) of claim 9, wherein each second receptor site mimic (22) comprises an artificial receptor site mimic of the second active agent (32).

11. The device (10) of claim 9 or claim 10, wherein each second receptor site mimic (22) is defined in the polymeric structure (20) by a molecular imprint of the second active agent (18).

12. The device (10) of any preceding claim, wherein the plurality of first receptor site mimics (22) has binding affinity for one or both of an anti-restenosis drug and an anti-thrombosis drug.

13. The device (10) of claim 7 or claim 8, wherein the first active agent (18) is selected from the group consisting of: noscapine, a microtubule stabilizer, paclitaxel, a taxane, a microtubule destabilizer, vincristine, vinblastine, podophylotoxin, estramustine, griseofulvin, dicoumarol, a vinca alkaloid, a heparin, a heparinoidm warfarin, an RGD peptide, aspirin, and any combination thereof.

14. The device (10) according to any of claims 9-12, wherein the first active agent (18) is selected from the group consisting of: noscapine, a microtubule stabilizer, paclitaxel, a taxane, a microtubule destabilizer, vincristine, vinblastine, podophylotoxin, estramustine, griseofulvin, dicoumarol, a vinca alkaloid, a heparin, a heparinoid, warfarin, an RGD peptide, aspirin, and any combination thereof.

15. The device (10) of any of claims 1-14, wherein an active agent (18) comprises noscapine.

16. The device (10) of any preceding claim, further comprising a plurality of said polymeric structures (20).

17. The device of claim 16, wherein said plurality of polymeric structures is adapted to repeatedly bind to, temporarily hold, and release an active agent (18).

18. The device (10) of any preceding claim, wherein a plurality of first receptor site mimics (22) includes receptor site mimics having varying binding affinities for an active agent (18).

## Patentansprüche

1. Implantierbare medizinische Vorrichtung (10), umfassend:
ein gestrecktes Element (12B), das als eine Vielzahl von zirkulären Windungen konfiguriert ist, die eine erste Helix bilden, wobei die ersten Helix einen inneren Hohlraum (14B) definiert, und wobei benachbarte Windungen der ersten Helix eine Öffnung (16B) definieren, die den inneren Hohlraum (14B) mit der Außenseite der Helix verbindet; und
eine polymere Struktur (20), die innerhalb des inneren Hohlraums (14) aufgebracht ist, **dadurch gekennzeichnet, dass** die polymere Struktur (20) eine Vielzahl von künstlichen ersten Rezeptorbindungsstellenmimetika (22) hat, und die Öffnung (16) ausreichend groß ist, um Blutzellen daran zu hindern in den inneren Hohlraum (14) zu gelangen.

2. Vorrichtung (10) nach Anspruch 1, wobei die erste Helix weiterhin konfiguriert ist, um eine zweite Helix (11B) zu bilden, wobei die zweite Helix einen mittig gelegenen Durchgang (13B) für einen Durchtritt von Fluid dorthindurch definiert.

3. Vorrichtung (10) nach Anspruch 1 oder Anspruch 2, wobei die Öffnung (16B) von einem Raum (D1) zwischen benachbarten Windungen der ersten Helix definiert wird.

4. Vorrichtung (10) nach Anspruch 3, wobei der Raum (D1) zwischen benachbarten Windungen der ersten Helix kleiner oder gleich ungefähr 8,0 Mikrons ist.

5. Vorrichtung (10) nach Anspruch 4, wobei der Raum (D1) zwischen benachbarten Windungen der ersten Helix kleiner als ungefähr 5 Mikrons ist.

6. Vorrichtung (10) nach Anspruch 5, wobei der Raum (D1) zwischen benachbarten Windungen der ersten Helix zwischen ungefähr 0,5 Mikrons und ungefähr 1,0 Mikrons ist.

7. Vorrichtung (10) nach einem der vorherigen Ansprüche, wobei die ersten Rezeptorbindungsstellenmimetika (22) jeweils konfiguriert sind, um einen ersten Wirkstoff (18) zu binden.

8. Vorrichtung (10) nach Anspruch 7, wobei die ersten Rezeptorbindungsstellenmimetika (22) in der polymeren Struktur (20) von einem molekularen Abdruck des Wirkstoffes (18) definiert sind.

9. Vorrichtung (10) nach einem der vorherigen Ansprüche, weiterhin umfassend eine Vielzahl von zweiten Rezeptorbindungsstellenmimetika (22), wobei jedes zweite Rezeptorbindungsstellenmimetikum (22) konfiguriert ist, einen zweiten Wirkstoff (32) zu binden.

10. Vorrichtung (10) nach Anspruch 9, wobei jedes Rezeptorbindungsstellenmimetikum (22) ein künstliches Rezeptorbindungsstellenmimetikum des zweiten Wirkstoffs (32) umfasst.

11. Vorrichtung (10) nach Anspruch 9 oder Anspruch 10, wobei jedes zweite Rezeptorbindungsstellenmimetikum (22) in der polymeren Struktur (20) von einem molekularen Abdruck des zweiten Wirkstoffs (18) definiert ist.

12. Vorrichtung (10) nach einem der vorherigen Ansprüche, wobei die Vielzahl von ersten Rezeptorbindungsstellenmimetika (22) eine Bindungsaffinität für einen Antirestenosewirkstoff oder einen Antithrombosewirkstoff oder für beide hat.

13. Vorrichtung (10) nach Anspruch 7 oder Anspruch 8, wobei der erste Wirkstoff (18) ausgewählt ist aus der Gruppe bestehend aus Noscapin, einem Microtubulistabilisator, Paclitaxel, einem Taxan, einem Mikrotubulidestabilisator, Vincristin, Vinblastin, Podophyllotoxin, Estramustin, Griseofulvin, Dicumarol, ein Vincaalkaloid, einem Heparin, einem Heparinoid, Warfarin, einem RGD-Peptid, Aspirin und beliebigen Kombinationen davon.

14. Vorrichtung (10) nach einem der Ansprüche 9 bis 12, wobei der erste Wirkstoff (18) ausgewählt ist aus der Gruppe bestehend aus Noscapin, einem Microtubulistabilisator, Paclitaxel, einem Taxan, einem Mikrotubulidestabilisator, Vincristin, Vinblastin, Podophyllotoxin, Estramustin, Griseofulvin, Dicumarol, ein Vincaalkaloid, einem Heparin, einem Heparinoid, Warfarin, einem RGD-Peptid, Aspirin und beliebigen Kombinationen davon.

15. Vorrichtung (10) nach einem der Ansprüche 1 bis 14, wobei der Wirkstoff (18) Noscapin umfasst.

16. Vorrichtung (10) nach einem der vorherigen Ansprüche, weiterhin umfassend eine Vielzahl der polymeren Strukturen (20).

17. Vorrichtung (10) nach Anspruch 16, wobei die Vielzahl von polymeren Strukturen ausgelegt sind, einen Wirkstoff (18) wiederholt zu binden, temporär zu halten und freizusetzen.

18. Vorrichtung (10) nach einem der vorherigen Ansprüche, wobei eine Vielzahl von ersten Rezeptorbindungsstellenmimetika (22) Rezeptorbindungstellenmimetika mit verschiedenen Bindungsaffinitäten für einen Wirkstoff (18) umfasst.

## Revendications

1. Dispositif médical implantable (10), comprenant :
un élément allongé (12B) configuré en une pluralité de tours circulaires formant une première hélice, la première hélice définissant une cavité interne (14B) et des tours adjacents de la première hélice définissant une ouverture (16B) raccordant la cavité interne (14B) avec l'extérieur de hélice ; et
une structure polymère (20) disposée dans la cavité interne (14), **caractérisée en ce que** ladite structure polymère (20) a une pluralité de premiers agents artificiels imitant un site de récepteur (22) et ladite ouverture (16) ayant une dimension suffisante pour empêcher que des cellules sanguines ne pénètrent dans la cavité interne (14).

2. Dispositif (10) selon la revendication 1, dans lequel la première hélice est en outre configurée de façon à former une deuxième hélice (11B), la deuxième hélice définissant un passage central (13B) pour le passage du fluide dans celui-ci.

3. Dispositif (10) selon la revendication 1 ou la revendication 2, dans lequel ladite ouverture (16B) est définie par un espace (D1) entre des tours adjacents de la première hélice.

4. Dispositif (10) selon la revendication 3, dans lequel l'espace (D1) entre des tours adjacents de la première hélice est inférieur ou égal à environ 8,0 microns.

5. Dispositif (10) selon la revendication 4, dans lequel l'espace (D1) entre les tours adjacents de la première hélice est inférieur à environ 5 microns.

6. Dispositif (10) selon la revendication 5, dans lequel l'espace (D1) entre les tours adjacents de la première hélice est compris entre environ 0,5 micron et environ 1,0 micron.

7. Dispositif (10) selon l'une quelconque des revendications précédentes, dans lequel les premiers agents imitant le site récepteur (22) sont configurés respectivement de façon à se lier à un premier agent actif (18).

8. Dispositif (10) selon la revendication 7, dans lequel les premiers agents imitant le site récepteur (22) sont définis dans la structure polymère (20) par une empreinte moléculaire de l'agent actif (18).

9. Dispositif (10) selon l'une quelconque des revendications précédentes, comprenant une pluralité de seconds agents imitant un site récepteur (22), chaque second agent imitant un site récepteur (22) étant configuré de façon à se lier à un deuxième agent actif (32).

10. Dispositif (10) selon la revendication 9, dans lequel chaque second agent imitant un site récepteur (22) comprend un agent imitant un site récepteur artificiel du deuxième agent actif (32).

11. Dispositif (10) selon la revendication 9 ou la revendication 10, dans lequel chaque second agent imitant le site récepteur (22) est défini dans la structure polymère (20) par une empreinte moléculaire du deuxième agent actif (18).

12. Dispositif (10) selon l'une quelconque des revendications précédentes, dans lequel la pluralité de premiers agents imitant un site récepteur (22) a une affinité de liaison pour un ou deux médicaments parmi un médicament anti-resténose et un médicament anti-thrombose.

13. Dispositif (10) selon la revendication 7 ou la revendication 8, dans lequel le premier agent actif (18) est choisi dans le groupe comprenant la noscapine, un stabilisant microtubulaire, le paclitaxel, un taxane, un déstabilisant microtubulaire, la vincristine, la vinblastine, la podophylotoxine, l'estramustine, la griséofulvine, le dicoumarol, un alcaloïde vinca, une héparine, un héparinoïde, la warfarine, un peptide RGD, de l'aspirine et toute combinaison de ceux-ci.

14. Dispositif (10) selon l'une quelconque des revendications 9 à 12, dans lequel le premier agent actif (18) est choisi dans le groupe comprenant la noscapine, un stabilisant microtubulaire, le paclitaxel, un taxane, un déstabilisant microtubulaire, la vincristine, la vinblastine, la podophylotoxine, l'estramustine, la griséofulvine, le dicoumarol, un alcaloïde vinca, une héparine, un héparinoïde, la warfarine, un peptide RGD, de l'aspirine et toute combinaison de ceux-ci.

15. Dispositif (10) selon l'une quelconque des revendications 1 à 14, dans lequel un agent actif (18) comprend la noscapine.

16. Dispositif (10) selon l'une quelconque des revendications précédentes, comprenant en outre une pluralité desdites structures polymères (20).

17. Dispositif selon la revendication 16, dans lequel ladite pluralité de structures polymères est adaptée pour se lier de façon répétitive, conserver temporairement et libérer un agent actif (18).

18. Dispositif (10) selon l'une quelconque des revendications précédentes, dans lequel une pluralité de premiers agents imitant un site récepteur (22) comprend des agents imitant un site récepteur ayant diverses affinités de liaison pour un agent actif (18).
